# FASCICULE DE BREVET EUROPEEN

(11) **EP 2 803 379 B1**
(45) Date de publication et mention de la délivrance du brevet: **17.02.2016**
(21) Numéro de dépôt: 14165356.8
(22) Date de dépôt: 22.04.2014
(51) Int. Cl.: A61M 16/00

(54) **Appareil de ventilation artificielle à capteur de pression absolue et capteur de pression différentielle**
Beatmungsgerät mit einem Absolutdrucksensor und einem Differenzdrucksensor
Apparatus for artificial ventilation having an absolute pressure sensor and a differential pressure sensor

(30) Priorité: 16.05.2013 FR 1354380
(43) Date de publication de la demande: 19.11.2014
(73) Titulaire: Air Liquide Medical Systems, 92160 Antony (FR)
(72) Inventeur: Boulanger, Thierry, 37000 Tours (FR); Colin, Clémence, 75009 Paris (FR); Libardi, Mickaël, 94270 Le Kremlin-Bicetre (FR)
(74) Mandataire: Pittis, Olivier

(56) Documents cités:
- WO-A2-2007/045017
- FR-A1- 2 956 819
- US-A1- 2006 283 450

## Description

L'invention concerne un appareil ou dispositif de ventilation artificielle comprenant, dans la branche inspiratoire du circuit patient, un capteur de pression absolue en redondance avec un capteur de pression différentielle.

Les patients en insuffisance respiratoire, par exemple ceux souffrant de broncho-pneumopathie chronique obstructive (BPCO), de syndrome de détresse respiratoire aigue (SDRA) ou bien ceux placés en service de soin post interventionnel, requièrent la mise en place d'une assistance respiratoire, c'est-à-dire qu'ils doivent être alimentés en gaz respiratoire, tel de l'air, de l'oxygène ou de l'air enrichi en oxygène, au moyen d'un appareil de ventilation artificielle.

Les appareils ou dispositifs de ventilation artificielle, couramment appelés respirateurs, régulent, dans certains modes de fonctionnement, la pression du gaz respiratoire à délivrer au patient. Cette régulation se fait grâce à un moyen de mesure de pression situé dans la branche inspiratoire du respirateur.

Une défaillance de ce moyen de mesure peut entraîner une régulation erronée aux conséquences sérieuses, voire dramatiques pour le patient, à savoir un barotraumatisme, c'est-à-dire la délivrance d'une pression trop élevée dans les poumons, donc au minimum dangereuse, voire létale pour le patient.

Afin de minimiser ou d'éviter ce risque, on agence communément dans le circuit patient des respirateurs deux capteurs de pression différentielle redondants, permettant de détecter une éventuelle panne de l'un d'eux, et prendre des mesures de réduction du risque. Cependant, cette solution engendre un surcoût important du fait de l'utilisation de plusieurs capteurs de pression différentielle.

Par ailleurs, nombre de respirateurs emploient un capteur de pression absolue permettant de mesurer la pression atmosphérique. Cette mesure de pression atmosphérique permet de compenser les autres moyens de mesure, notamment de débit, dont le comportement est impacté par la pression atmosphérique. A défaut de compensation, des volumes erronés seraient délivrés au patient, avec des conséquences parfois dramatiques. Ce moyen de mesure confère donc une mobilité en altitude au respirateur, mais engendre également un surcoût notable.

En outre, un capteur de pression absolue mesure habituellement la pression atmosphérique soit dans le boîtier du ventilateur, soit à l'extérieur du ventilateur. Or, une défaillance de ce moyen de mesure conduisant à une valeur erronée de la mesure ne peut être détectée que lors d'une intervention humaine, nécessitant potentiellement d'ouvrir le boîtier. Pour y remédier et éviter cette intervention fastidieuse, il faut doubler ce moyen de mesure, ce qui engendre également un surcoût notable.

Il s'ensuit qu'avec les respirateurs actuels, plusieurs problèmes se posent simultanément, à savoir :
- de disposer d'une mesure de pression différentielle et d'une mesure de pression atmosphérique et d'un moyen de détecter une panne sur l'un ou l'autre des moyens de mesure sans intervention humaine et sans engendrer de surcoût.
- de permettre un étalonnage aisé avec deux valeurs de référence de ce capteur, sans moyen supplémentaire de pressurisation / dépressurisation. En effet, les capteurs de pression absolue étant généralement affines, c'est-à-dire que le signal de sortie est une fonction affine du phénomène observé (la pression absolue), leur étalonnage nécessite deux valeurs de pression de référence. Des moyens supplémentaires sont donc nécessaires pour appliquer sur le capteur une surpression ou une dépression par rapport à la pression atmosphérique, afin d'obtenir une deuxième pression de référence.
- de rendre possible la mesure de dépressions engendrées par le patient, sans nuire à la qualité de la mesure des pressions relatives usuellement positives, et sans surcoût notable. En effet, certaines fonctionnalités des respirateurs, telle la mesure de la Force Inspiratoire Négative (*Negative Inspiratory Force* ou *NIF*)*,* nécessitent de mesurer la dépression engendrée par l'effort musculaire (respiratoire) du patient. Les valeurs de dépression à considérer sont typiquement de l'ordre de -30 à -100 cm H₂0. Or, usuellement, les capteurs de pression différentielle équipant les respirateurs ne mesurent pas les pressions relatives négatives, du moins pas pour de telles dépressions. Une solution consisterait à utiliser des capteurs adaptés à la mesure de telles dépressions, mais puisque leur intervalle de mesure serait plus large, leur résolution serait amoindrie et ce, aux dépens de la qualité de la régulation en pression.

Le document WO-A-2007/045017 enseigne un dispositif de délivrance de gaz permettant de fournir un gaz humidifié à une personne. Il comprend une branche inspiratoire équipée d'un capteur de pression et d'un capteur de débit reliés à une unité centrale de commande (CPU).

Le document FR-A-2956819 propose un dispositif de suivi de l'observance d'un traitement de l'apnée du sommeil comprenant une branche inspiratoire équipée d'un capteur de pression relative ou de deux capteurs de pression absolue reliés à des moyens de traitement d'information. Les données recueillies sont stockées dans des moyens de mémorisation, puis émises vers un serveur disatnt, par un système d'émission.

Le document US-A-2006/283450 décrit un système de fourniture de gaz comprenant une source de gaz sous pression alimentant une branche inspiratoire équipée d'un capteur de pression et d'un capteur de débit reliés à un module de traitement de données de manière à commander une valve de contrôle du passage de gaz agencée dans la branche inspiratoire.

La présente invention vise à proposer un respirateur amélioré ne présentant pas tout ou partie des problèmes ci-dessus.

La solution est alors un appareil de ventilation artificielle comprenant un circuit patient véhiculant du gaz comprenant une branche inspiratoire, et au moins un capteur de pression agencé de manière à opérer au moins une mesure de pression dans le circuit patient, caractérisé en ce qu'il comprend :
- un capteur de pression différentielle et un capteur de pression absolue agencés sur la branche inspiratoire, et
- des moyens de pilotage coopérant avec le capteur de pression différentielle et le capteur de pression absolue pour détecter une panne ou un dysfonctionnement de l'un ou l'autre desdits capteurs de pression différentielle et de pression absolue.

Selon le cas, l'appareil de l'invention peut comprendre l'une ou plusieurs des caractéristiques techniques suivantes :
- le capteur de pression différentielle et le capteur de pression absolue sont agencés en série.
- la branche expiratoire ne comporte aucun capteur de pression.
- le circuit patient comprend une branche inspiratoire et une branche expiratoire.
- la branche inspiratoire et la branche expiratoire sont reliées l'une à l'autre par une pièce en Y.
- la pièce en Y est elle-même reliée à une interface patient, en particulier un masque respiratoire, une sonde trachéale ou tout autre dispositif d'administration de gaz à un patient.
- les moyens de pilotage sont aptes à et conçus pour détecter une panne ou un dysfonctionnement de l'un ou l'autre desdits capteurs de pression différentielle et de pression absolue à partir d'une ou plusieurs valeurs de pression absolue (Pabs) mesurées par le capteur de pression absolue et de pression différentielle (Pdiff) mesurées par le capteur de pression différentielle.
- les moyens de pilotage sont aptes à et conçus pour déterminer une ou plusieurs valeurs de pression atmosphérique (Patm) à partir d'une ou plusieurs valeurs de pression absolue (Pabs) mesurées par le capteur de pression absolue et d'en déduire une valeur de pression différentielle (Pdiff_B) ainsi que d'une ou plusieurs valeurs de pression différentielle (Pdiff) mesurées par le capteur de pression différentielle.
- les moyens de pilotage sont aptes à et conçus pour détecter toute variation rapide de l'écart entre les valeurs de pression (Pdiff_B) issue du capteur de pression absolue et (Pdiff) issue du capteur de pression différentielle.
- les moyens de pilotage sont aptes à et conçus pour détecter toute variation significative et rapide de l'écart entre les valeurs de pression (Pdiff_B) issue du capteur de pression absolue (5) et (Pdiff) issue du capteur de pression différentielle (4), ladite variation étant significative et rapide lorsqu'elle est supérieure ou égale à une valeur de variation de pression préfixée (Dp) pendant une durée (t) donnée. Ainsi, on peut considérer qu'une valeur de variation de pression préfixée Dp égale à 5mb ou plus, entre Pdiff et Pdiff_B dans la plage de mesure couverte par Pdiff est jugée significative, et qu'une telle différence observée sur une durée t comprise entre 100 ms à 10 s est jugée rapide au regard des variations naturelles de la pression atmosphérique.
- les moyens de pilotage comprennent une carte électronique à microcontrôleur.
- les moyens de pilotage comprennent une carte électronique à microcontrôleur qui recueille les signaux électriques des capteurs de pression absolue et différentielle afin d'opérer différents traitements permettant d'obtenir les valeurs de pression susmentionnées.
- il comprend une carte électronique à microcontrôleur utilisant valeur de pression Pdiff pour la comparer à la pression de consigne à appliquer au patient, ladite carte électronique pilotant la valve inspiratoire agencée au niveau de la branche inspiratoire pour l'ouvrir ou la fermer afin que les mesures de Pdiff et de la pression de consigne soient égales (ou sensiblement égales) à chaque instant.
- les moyens de pilotage comprennent un filtre passe-bas. il comprend au moins une source de gaz délivrant un gaz choisi parmi l'oxygène et l'air, de préférence au moins une source de gaz est une bouteille de gaz ou une canalisation de gaz débouchant au niveau d'une prise de raccordement, en particulier une prise murale de distribution de gaz à laquelle est relié fluidiquement l'appareil.
- il comprend au moins une turbine alimentant la branche inspiratoire en gaz, de préférence en un gaz issu d'au moins une source de gaz.
- il comprend une valve inspiratoire agencée au niveau de la branche inspiratoire, ladite valve inspiratoire coopérant avec le capteur de pression différentielle de manière à permettre par exemple la délivrance d'une alternance de deux niveaux de pression au patient selon que l'on considère une phase inspiratoire ou bien une phase expiratoire
- la valve inspiratoire est agencée sur la branche inspiratoire, entre la turbine et les capteurs de pression différentielle et de pression absolue.

La présente invention va maintenant être décrite plus en détail en références aux Figures annexées parmi lesquelles :
- la Figure 1 est un schéma de principe d'un mode de réalisation d'un respirateur selon la présente invention et
- la Figure 2 est un synoptique illustrant les mesures de pression et de détection de panne.

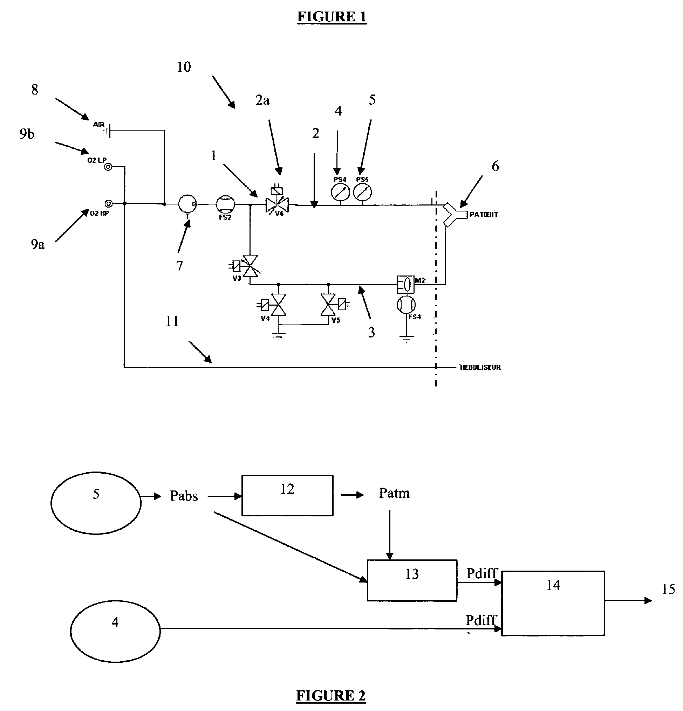

La Figure 1 représente le schéma de principe d'un appareil de ventilation artificielle ou plus simplement respirateur 10 selon la présente invention.

Ce respirateur 10 comprend un circuit patient 1 comprenant des passages de gaz permettant d'acheminer le gaz à administrer au patient depuis une ou plusieurs sources de gaz 8, 9a, 9b ou, à l'inverse, récupérer les gaz expirés par le patient.

Le circuit patient 1 comprend en particulier une branche inspiratoire 2 et une branche expiratoire 3 formant un circuit en boucle. Ces branches inspiratoire 2 et expiratoire 3 sont reliées l'une à l'autre au niveau d'une pièce en Y 6 située au niveau de l'interface patient, c'est-à-dire d'un masque, une sonde trachéale ou de tout autre dispositif d'administration de gaz respiratoire au patient. Toutefois, de façon alternative, le circuit patient 1 pourrait ne comprendre qu'une branche inspiratoire 2, c'est-à-dire être dépourvu de toute branche expiratoire.

Selon la présente invention, un capteur de pression différentielle 4 et un capteur de pression absolue 5 sont agencés sur la branche inspiratoire 2 du respirateur 11, c'est-à-dire que ces capteurs 4, 5 sont tous les deux reliés pneumatiquement à la branche inspiratoire 2 du respirateur de manière à y opérer des mesures de pression. Par contre, selon l'invention, aucun capteur n'est agencé sur la branche expiratoire 3.

Le gaz respiratoire circule au sein du circuit patient 1, en particulier dans la branche inspiratoire 2, au moyen d'une turbine 7 agencé en amont de la branche inspiratoire 2 dans laquelle est également placée une valve inspiratoire 2a.

Le gaz respiratoire est de l'air issu d'une source d'air 8, tel l'atmosphère ambiante, de l'oxygène issu d'une source d'oxygène à haute pression 9a (typiquement > 3 bars) ou à basse pression 9b (typiquement < 1 bar), ou de l'air enrichi en oxygène obtenu par mélange d'air et d'oxygène issus desdites sources 8, 9a ou 9b. Ce gaz, comprimé par la turbine 7, est mis en forme par la valve inspiratoire 2a afin d'assurer la respiration du patient.

Dans le cadre d'une ventilation barométrique où une pression intermittente fixe est générée à la bouche du patient, la carte microcontrôleur utilise la valeur transformée Pdiff, issue du capteur de pression différentielle 4, pour la comparer à la pression de consigne à appliquer au patient et piloter en conséquence la valve inspiratoire 2a, c'est-à-dire progressivement l'ouvrir ou inversement la fermer afin que les mesures de Pdiff et de la pression de consigne soient égales à chaque instant.

Comme détaillé ci-après, selon l'invention, les valeurs de pression mesurées par les capteurs 4, 5 sont traités par des moyens de pilotage qui comprennent une carte électronique à microcontrôleur, pour détecter une panne ou un dysfonctionnement de l'un ou l'autre desdits capteurs de pression différentielle 4 et de pression absolue 5, et ensuite alerter l'utilisateur de cette panne ou dysfonctionnement.

L'appareil 10 comprend en outre une ligne de gaz supplémentaire 11 reliée fluidiquement aux sources d'oxygène à haute pression 9a et à basse pression 9b pour permettre d'acheminer de l'oxygène jusqu'à un dispositif de nébulisation pouvant être raccordé à ladite ligne supplémentaire 11.

Il est à noter que les lignes d'admission ou d'amenée de gaz peuvent comprendre en outre des éléments supplémentaires (non montrés en Figure 1), tel que des filtres, clapets anti retour, moyens de mesure de débit, de pression, une ou des électrovannes... qui permettent de contrôler l'amenée et la circulation des gaz au sein du dispositif.

La Figure 2 est un synoptique schématisant les mesures de pression différentielle et de pression atmosphérique opérées respectivement par les capteur de pression différentielle 4 et capteur de pression absolue 5 agencés sur la branche inspiratoire 2, et l'utilisation de ces mesures pour détecter une panne éventuelle de l'un ou l'autre des capteurs 4 et 5.

Le capteur de pression absolue 5 mesure la somme Pabs de la pression atmosphérique Patm et de la pression différentielle Pdiff, Pdiff étant par ailleurs mesurée avec le capteur de pression différentielle 4, c'est-à-dire que l'on a : Pabs = Patm + Pdiff

En général, la pression atmosphérique varie lentement, pour des raisons climatiques ou de transport, par exemple lors d'un changement d'altitude.

Il est donc possible, en filtrant Pabs au sein d'un filtre passe-bas 12, de déterminer la part liée à la pression atmosphérique Patm et celle liée à la pression différentielle Pdiff, c'est-à-dire, autrement dit, de reconstruire une mesure de pression différentielle Pdiff_B issue de la seule mesure du capteur de pression absolue 5. Cette mesure est possible en soustrayant (en 13) les valeurs Pabs et Patm issue du filtre passe bas 12.

Cette mesure Pdiff_B, reconstruite, peut alors être comparée à la valeur de Pdiff issue de 4 : toute variation significative et rapide, de l'écart (14) entre Pdiff et Pdiff_B est le signe d'une défaillance et non d'une variation naturelle de pression atmosphérique. Par exemple, et sans s'y limiter, on peut considérer qu'une différence de pression donnée, par exemple de 5mb entre Pdiff et Pdiff_B dans la plage de mesure couverte par Pdiff, est jugée significative et qu'une telle différence observée sur une échelle de temps de 100 ms à 10 s est jugée rapide au regard des variations naturelles de la pression atmosphérique.

Ceci permet ainsi de détecter en temps réel une panne ou un dysfonctionnement de l'un ou l'autre des capteurs 4, 5, sans intervention humaine et sans engendrer de surcoût. En cas de défaillance détectée (en 15), une alarme alerte l'utilisateur.

Par ailleurs, grâce aux deux capteurs 4, 5 agencés dans la branche inspiratoire 2 selon la présente invention, on peut facilement opérer à l'étalonnage, du capteur de pression absolue 5 sans moyen supplémentaire de pressurisation / dépressurisation (ce qui serait le cas si le capteur de pression absolue était situé dans le boîtier du ventilateur en dehors du schéma pneumatique).

Lorsque le capteur 4 pression différentielle Pdiff est lui-même étalonné et que la pression atmosphérique est connue, on peut alors opérer selon les étapes suivantes :
- étape a) : lorsque la pression du respirateur 10 est coupée (turbine 7 arrêtée, valve inspiratoire 2a fermée...), le capteur de pression atmosphérique 5 mesure la pression atmosphérique (Pabs), ce qui donne un premier point de référence.
- étape b) : au moyen du respirateur 10 (turbine 7, valve inspiratoire 2a...) et du capteur 4 pression différentielle Pdiff, on régule une pression différentielle dans la branche inspiratoire 2 du circuit patient 1.
- étape c) : le capteur 5 de pression absolue Pabs mesure ainsi la somme des pressions atmosphérique et différentielle, ce qui donne un deuxième point de référence,
- étape d) : on détermine la fonction affine à partir desdits deux points de référence sans qu'il n'y ait nécessité à utiliser un moyen de pressurisation et dépressurisation particulier.

Enfin, la mesure de dépression étant ponctuelle dans le cadre de la NIF, toute variation négative et rapide de la pression Pabs est signe d'une dépression. L'amplitude de cette variation est l'amplitude de la dépression.

Ceci rend possible la mesure des dépressions engendrées par le patient, sans nuire à la qualité de la mesure des pressions relatives usuellement positives, et sans surcoût notable.

L'appareil de ventilation artificielle dont la branche inspiratoire du circuit patient véhicule un gaz respiratoire et comprend, en outre, un capteur de pression différentielle et un capteur de pression absolue est utilisable dans une méthode de traitement thérapeutique d'un patient requérant une ventilation assistée au moyen d'un tel appareil, en particulier ceux souffrant d'une broncho-pneumopathie chronique obstructive (BPCO), d'un syndrome de détresse respiratoire aigue (SDRA) ou bien ceux placés en service de soin post interventionnel.

## Revendications

1. Appareil de ventilation artificielle comprenant un circuit patient (1) véhiculant du gaz comprenant au moins une branche inspiratoire (2), et au moins un capteur de pression agencé de manière à opérer au moins une mesure de pression dans le circuit patient (1), **caractérisé en ce qu'**il comprend :
- un capteur de pression différentielle (4) et un capteur de pression absolue (5) agencés sur ladite branche inspiratoire (2), et
- des moyens de pilotage coopérant avec le capteur de pression différentielle (4) et le capteur de pression absolue (5) pour détecter une panne ou un dysfonctionnement de l'un ou l'autre desdits capteurs de pression différentielle (4) et de pression absolue (5).

2. Appareil selon la revendication précédente, **caractérisé en ce que** le capteur de pression différentielle (4) et le capteur de pression absolue (5) sont agencés en série dans la branche inspiratoire (2).

3. Appareil selon l'une des revendications précédentes, **caractérisé en ce que** la branche expiratoire (3) ne comporte aucun capteur de pression.

4. Appareil selon l'une des revendications précédentes, **caractérisé en ce que** le circuit patient (1) comprend au moins une branche inspiratoire (2) et une branche expiratoire (3).

5. Appareil selon l'une des revendications précédentes, **caractérisé en ce que** la branche inspiratoire (2) et la branche expiratoire (3) sont reliées l'une à l'autre par une pièce en Y (6).

6. Appareil selon l'une des revendications précédentes, **caractérisé en ce que** les moyens de pilotage sont aptes à et conçus pour détecter une panne ou un dysfonctionnement de l'un ou l'autre desdits capteurs de pression différentielle (4) et de pression absolue (5) à partir d'une ou plusieurs valeurs de pression absolue (Pabs) mesurées par le capteur de pression absolue (5) et de pression différentielle (Pdiff) mesurées par le capteur de pression différentielle (4).

7. Appareil selon l'une des revendications précédentes, **caractérisé en ce que** les moyens de pilotage sont aptes à et conçus pour déterminer :
- une ou plusieurs valeurs de pression atmosphérique (Patm) à partir d'une ou plusieurs valeurs de pression absolue (Pabs) mesurées par le capteur de pression absolue (5) et d'en déduire une valeur de pression différentielle (Pdiff_B) et
- une ou plusieurs valeurs de pression différentielle (Pdiff) mesurées par le capteur de pression différentielle (4).

8. Appareil selon l'une des revendications précédentes, **caractérisé en ce que** les moyens de pilotage sont aptes à et conçus pour détecter toute variation significative et rapide de l'écart entre les valeurs de pression (Pdiff_B) issue du capteur de pression absolue (5) et (Pdiff) issue du capteur de pression différentielle (4), ladite variation étant significative et rapide lorsqu'elle est supérieure ou égale à une valeur de variation de pression préfixée (Dp) pendant une durée (t) donnée.

9. Appareil de ventilation artificielle selon l'une des revendications précédentes, **caractérisé en ce que** les moyens de pilotage comprennent une carte électronique à microcontrôleur.

10. Appareil de ventilation artificielle selon l'une des revendications précédentes, **caractérisé en ce que** les moyens de pilotage comprennent un filtre passe-bas (12).

11. Appareil selon l'une des revendications précédentes, **caractérisé en ce qu'**il comprend au moins une source de gaz (8, 9a, 9b) délivrant un gaz choisi parmi l'oxygène et l'air.

12. Appareil selon l'une des revendications précédentes, **caractérisé en ce qu'**il comprend au moins une turbine (7) alimentant en gaz la branche inspiratoire (2).

13. Appareil selon l'une des revendications précédentes, **caractérisé en ce qu'**il comprend une carte électronique à microcontrôleur utilisant valeur de pression Pdiff pour la comparer à la pression de consigne à appliquer au patient, ladite carte électronique pilotant une valve inspiratoire (2a) agencée au niveau de la branche inspiratoire (2), pour l'ouvrir ou la fermer afin que les mesures de Pdiff et de la pression de consigne soient égales à chaque instant.

## Patentansprüche

1. Gerät zur künstlichen Beatmung, einen Patientenkreislauf (1) zur Beförderung des Gases umfassend, der zumindest einen inspiratorischen Zweig (2) umfasst, und zumindest einen Drucksensor, der angeordnet ist, um zumindest eine Druckmessung im Patientenkreislauf (1) durchzuführen, **dadurch gekennzeichnet, dass** es folgendes umfasst:
- einen Differenzialdrucksensor (4) und einen Absolutdrucksensor (5), die auf dem besagten inspiratorischen Zweig (2) angeordnet sind, und
- Steuermittel, die mit dem Differenzialdrucksensor (4) und dem Absolutdrucksensor (5) zusammenwirken, um eine Panne oder eine Funktionsstörung des einen oder des anderen der besagten Sensoren für Differenzialdruck (4) und Absolutdruck (5) zu erfassen.

2. Gerät nach dem vorherigen Anspruch, **dadurch gekennzeichnet, dass** der Differenzialdrucksensor (4) und der Absolutdrucksensor (5) in Reihe im inspiratorischen Zweig (2) angeordnet sind.

3. Gerät nach einem der vorherigen Ansprüche, **dadurch gekennzeichnet, dass** der exspiratorische Zweig (3) keinen Drucksensor umfasst.

4. Gerät nach einem der vorherigen Ansprüche, **dadurch gekennzeichnet, dass** der Patientenkreislauf (1) zumindest einen inspiratorischen Zweig (2) und einen exspiratorischen Zweig (3) umfasst.

5. Gerät nach einem der vorherigen Ansprüche, **dadurch gekennzeichnet, dass** der inspiratorische Zweig (2) und der exspiratorische Zweig (3) durch ein Y-förmiges Stück (6) miteinander verbunden sind.

6. Gerät nach einem der vorherigen Ansprüche, **dadurch gekennzeichnet, dass** die Steuermittel in der Lage und gestaltet sind, um eine Panne oder eine Funktionsstörung des einen oder des anderen der besagten Sensoren für Differenzialruck (4) und Absolutdruck (5) ausgehend von einem oder mehreren Werten für Absolutdruck (Pabs) zu erfassen, die vom Absolutdrucksensor (5) gemessen werden, und für Differenzialdruck (Pdiff), die vom Differenzialdrucksensor (4) gemessen werden.

7. Gerät nach einem der vorherigen Ansprüche, **dadurch gekennzeichnet, dass** die Steuermittel in der Lage und gestaltet sind, um folgendes zu bestimmen:
- einen oder mehrere atmosphärische Druckwerte (Patm) ausgehend von einem oder mehreren Absolutdruckwerten (Pabs), die vom Absolutdrucksensor (5) gemessen werden, und um daraus einen Differenzialdruckwert (Pdiff_B) abzuleiten und
- einen oder mehrere Differenzialdruckwerte (Pdiff), die vom Differenzialdrucksensor (4) gemessen werden.

8. Gerät nach einem der vorherigen Ansprüche, **dadurch gekennzeichnet, dass** die Steuermittel in der Lage und gestaltet sind, um jede signifikante und rasche Variation der Abweichung zwischen den Druckwerten (Pdiff_B) aus dem Absolutdrucksensor (5) und (Pdiff) aus dem Differenzialdrucksensor (4) zu erfassen, wobei die besagte Variation signifikant und rasch ist, wenn sie größer oder gleich einem vorbestimmten Druckvariationswert (Dp) während einer gegebenen Zeitdauer (t) ist.

9. Gerät zur künstlichen Beatmung nach einem der vorherigen Ansprüche, **dadurch gekennzeichnet, dass** die Steuermittel eine Elektronikkarte mit Microcontroller umfassen.

10. Gerät zur künstlichen Beatmung nach einem der vorherigen Ansprüche, **dadurch gekennzeichnet, dass** die Steuermittel einen Niederpassfilter (12) umfassen.

11. Gerät nach einem der vorherigen Ansprüche, **dadurch gekennzeichnet, dass** es zumindest eine Gasquelle (8, 9a, 9b) umfasst, die ein aus Sauerstoff und Luft ausgewähltes Gas abgibt.

12. Gerät nach einem der vorherigen Ansprüche, **dadurch gekennzeichnet, dass** es zumindest eine Turbine (7) umfasst, die den inspiratorischen Zweig (2) mit Gas versorgt.

13. Gerät nach einem der vorherigen Ansprüche, **dadurch gekennzeichnet, dass** es eine Elektronikkarte mit Microcontroller umfasst, die den Druckwert Pdiff verwendet, um ihn mit der Druckvorgabe zu vergleichen, die auf den Patienten anzuwenden ist, wobei die besagte Elektronikkarte ein Inspirationsventil (2a) ansteuert, das im Bereich des inspiratorischen Zweigs (2) angeordnet ist, um es zu öffnen oder es zu schließen, damit die Messwerte von Pdiff und der Druckvorgabe zu jedem Zeitpunkt gleich sind.

## Claims

1. Apparatus for artificial ventilation comprising a patient circuit (1) transporting gas, comprising at least one inspiratory branch (2), and at least one pressure sensor, arranged so as to operate at least one pressure measurement within the patient circuit (1), **characterised in that** it comprises:
- a differential pressure sensor (4) and an absolute pressure sensor (5) arranged on said inspiratory branch (2), and
- control means engaging with the differential pressure sensor (4) and the absolute pressure sensor (5) to detect a failure or malfunction of one or the other of said differential pressure sensor (4) and absolute pressure sensor (5).

2. Apparatus according to the previous claim, **characterised in that** the differential pressure sensor (4) and the absolute pressure sensor (5) are arranged in series in the inspiratory branch (2).

3. Apparatus according to one of the previous claims, **characterised in that** the expiratory branch (3) does not include any pressure sensors.

4. Apparatus according to one of the previous claims, **characterised in that** the patient circuit (1) comprises at least one inspiratory branch (2) and one expiratory branch (3).

5. Apparatus according to one of the previous claims, **characterised in that** the inspiratory branch (2) and the expiratory branch (3) are connected to each other via a Y-shaped part (6).

6. Apparatus according to one of the previous claims, **characterised in that** the control means are capable of and designed to detect a failure or a malfunction of one or the other of said differential pressure sensor (4) and absolute pressure sensor (5) based on one or more absolute pressure values (Pabs) measured by the absolute pressure sensor (5) and differential pressure values (Pdiff) measured by the differential pressure sensor (4).

7. Apparatus according to one of the previous claims, **characterised in that** the control means are capable of and designed to determine:
- one or more atmospheric pressure values (Patm) based on one or more absolute pressure values (Pabs) measured by the absolute pressure sensor (5) and deduce therefrom a differential pressure value (Pdiff_B) and
- one or more differential pressure values (Pdiff) measured by the differential pressure sensor (4).

8. Apparatus according to one of the previous claims, **characterised in that** the control means are capable of and designed to detect any significant and fast variation in the difference between the pressure values (Pdiff_B) derived from the absolute pressure sensor (5) and (Pdiff) derived from the differential pressure sensor (4), said variation being significant and fast when it is greater than or equal to a pre-set pressure variation value (Dp) over a given period of time (t).

9. Artificial ventilation apparatus according to one of the previous claims, **characterised in that** the control means include a microcontroller circuit board.

10. Artificial ventilation apparatus according to one of the previous claims, **characterised in that** the control means include a low-pass filter (12).

11. Apparatus according to one of the previous claims, **characterised in that** it comprises at least one gas source (8, 9a, 9b) supplying a gas selected from oxygen and air.

12. Apparatus according to one of the previous claims, **characterised in that** it comprises at least one turbine (7) feeding the inspiratory branch (2) with gas.

13. Apparatus according to one of the previous claims, **characterised in that** it comprises a microcontroller circuit board using the pressure value Pdiff for comparison with the setpoint pressure to be applied to the patient, said circuit board controlling an inspiratory valve (2a) arranged at the level of the inspiratory branch (2), to open or close the latter so that the Pdiff and setpoint pressure measurements are equal at all times.
